# EUROPEAN PATENT APPLICATION

(11) **EP 2 505 181 A2**
(43) Date of publication of application: **03.10.2012**
(21) Application number: 12162751.7
(22) Date of filing: 30.03.2012
(51) Int. Cl.: A61K 8/06, A61K 8/31, A61K 8/34, A61K 8/81, A61K 8/92, A61Q 1/14, A61Q 19/10, A61Q 19/00

(54) **Creamy cleansing compositions**

(30) Priority: 30.03.2011 US 201113075767
(71) Applicant: Johnson & Johnson Consumer Companies Inc., Skillman, NJ 08558 (US)
(72) Inventor: Cossa, Anthony J., Branchburg, NJ New Jersey 08876 (US); Diamond, John, Plymouth Meeting, PA Pennsylvania 19462 (US); Santora, Delores, Ringoes, NJ New Jersey 08551 (US); Walters, Russel, Philadelphia, PA Pennsylvania 19106 (US)
(74) Representative: Kirsch, Susan Edith

(57) **Abstract**

A creamy cleansing composition including from about 0.5 % to about 1.2 % polymeric emulsifier; from about 0.5 % to about 4 % water insoluble oil; from about 1 % to about 4 % fatty alcohol; and greater than 92 % water is described.

## Description

### FIELD OF THE INVENTION

The compositions of this invention relate to creamy cleansing compositions that provide excellent cleansing, mildness and a smooth skin feel.

### BACKGROUND OF THE INVENTION

Skin cleansers should ideally be very mild to the skin and provide excellent removal of unwanted material. However, often, mild cleansing formulations can be less effective in removing dirt than compositions that may be harsher to the skin. In cleansers, surfactants are used to help remove unwanted materials, such as dirt, oils, bacteria and the like, from the skin. The surfactants act by forming micellar structures with unwanted material which bring the unwanted material into the aqueous phase to be washed off.

Typically good cleansing requires some sufficiently high level of surfactant. High levels of surfactant are associated with irritation and skin barrier damage. As the surfactant level in a cleanser is increased, the foaming and cleansing ability increase and there is a corresponding decrease in the cleanser mildness. It has long been desired in the art to have a high cleansing and mild formula. However, cleansing systems containing high levels of surfactant are often harsh or aggressive to the skin. Such cleansing systems typically provide excellent cleansing, while less aggressive cleansing systems that are mild to the skin provide only limited or reduced cleansing.

Creamy cleansing compositions are commercially available. The compositions typically are opaque and include an oil phase and surfactants to aid in dissolving dirt and an emollient to be deposited on the skin and provide a smooth skin feel. The compositions tend to have deficiencies in that they are not effective cleansers, are not mild, or do not provide an excellent smooth skin feel. There are creamy cleansers that are largely devoid of surfactant, that are mild to the skin, but in general, these compositions do not provide sufficient cleansing. There are also creamy cleansers that exist that have higher surfactant loads, that provide sufficient cleansing and foaming, but typically these are not mild to the skin. Therefore, there is a need for a creamy cleansing composition that provides excellent cleansing, mildness, and a smooth skin feel.

### SUMMARY OF THE INVENTION

The compositions of this invention relate to creamy cleansing compositions comprising, consisting essentially of and consisting of the following ingredients: from about 0.5 % to about 1.2 % polymeric emulsifier; from about 0.5 % to about 4 % water insoluble oil; from about 1 % to about 4 % fatty alcohol; and greater than 92 % water.

### DETAILED DESCRIPTION OF THE INVENTION

Compositions according to this invention preferably include a polymeric emulsifier. As used herein, the term "polymeric emulsifier" refers to a polymer having both hydrophilic and hydrophobic moieties that is capable of contributing to the formation of a stable emulsion between an oil phase and an aqueous phase. Typically, a stable emulsion is an emulsion that shows no phase separation on standing for one week, as determined by the unaided human eye. Any of a variety of suitable polymeric emulsifiers may be used in the compositions of this invention. Polymeric emulsifiers may be useful in providing shelf stability to the composition into which they are added. In addition, polymeric emulsifiers may be useful in facilitating the deposition of hydrophobic agent onto a substrate. They also act to support the ability of the composition to provide foam, such as when the compositions of this invention are used to cleanse skin. In addition, more preferably, polymeric emulsifiers are chosen from those compounds that are water-soluble and capable of forming a phase-stable emulsion. Preferably, such emulsions remain stable for at least about one week, more preferably for at least about one month, with a hydrophobic agent in water.

As used herein a material is defined as "water-soluble" if it forms a clear solution that is stable at room temperature (no settling or phase-instability) for 48 hours by adding only 0.5% by weight of such material in deionized water.

Polymeric emulsifiers useful in the compositions of this invention are preferably salt-sensitive, meaning that their solubility in water is reduced, often dramatically, in the presence of electrolytes. Electrolytes may be present as impurities in surfactant cleansing systems and on the surface of skin. A polymeric emulsifier is defined as "salt-sensitive" if it loses its ability to remain phase -stable in aqueous solution when sodium chloride has been added to the solution. More specifically, a "salt sensitive" polymeric emulsifier shows phase separation and/or a 30% or more change in viscosity, measured using a Brookfield viscometer with an LVT2 spindle at 12 RPM, when 3% sodium chloride is added to a homogenous solution of 1% (active) polymeric emulsifier in deionized water, wherein the viscosity is measured at ambient temperature.

The polymeric emulsifiers useful in the compositions of this invention may be of any suitable molecular weight. Polymeric emulsifiers suitable for use in the compositions of this invention may be an associative polymer, i.e., a polymer formed from monomers such that individual repeat units are hydrophilic, such as may be formed by addition polymerization of such as acids as acrylic, methacrylic, maleic, itaconic, and the like or by combination to form copolymers thereof. Other suitable polymeric emulsifiers include naturally-derived polymers such as polysaccharides that may be derivatized with hydrophobic moieties. Suitable hydrophilic groups include hydroxyl, carboxyl, sulphate, sulphonate, taurate, phosphates, phosphonates, amides, amines and the like. In order to provide hydrophobic character, such polymers may at least partially hydrophobically modified, such as by copolymerizing the polymer with hydrocarbons having a carbon chain length of at least about three carbon atoms.

Notable commercially available polymeric emulsifiers include, but are not limited to, hydrophobically modified polyacrylic acid commercially under the tradename Pemulen® TR-1 and TR-2 by Noveon, Inc., water-soluble or water-swellable copolymers based on acrylamidoalkyl sulfonic acid and cyclic N-vinylcarboxamides commercially available under the tradename Aristoflex® AVC by Clariant Corporation; water-soluble or water-swellable copolymers based on acrylamidoalkyl sulfonic acid and hydrophobically modified methacrylic acid commercially available under the tradename Aristoflex® HMB by Clariant Corporation and a homopolymer of acrylamidoalkyl sulfonic acid commercially available under the tradename Granthix APP by Grant Industries, Inc.

Another class of notable polymeric emulsifier includes hydrophobically-modified, crosslinked, anionic acrylic copolymers, including random polymers, but may also exist in other forms such as block, star, graft, and the like. The hydrophobically modified, crosslinked, anionic acrylic copolymer may be synthesized from at least one acidic monomer and at least one hydrophobic ethylenically unsaturated monomer. Examples of suitable acidic monomers include those ethylenically unsaturated acid monomers that may be neutralized by a base. Examples of suitable hydrophobic ethylenically unsaturated monomers include those that contain a hydrophobic chain having a carbon chain length of at least about three carbon atoms.

Other suitable polymeric emulsifiers include modified cellulose polymers such as those modified cellulose polymers described by the trade name KLUCEL, available from Hercules Corporation of Wilmington, DE. For example, hydroxyalkylcellulose materials are useful in the compositions of this invention. More preferably, they can be hydroxyethylcellulose, hydroxypropylcellulose, combinations thereof and the like.

The amount of polymeric emulsifier used in the compositions of this invention preferably contain from about 0.5 weight percent to about 1.2 weight percent of the composition. More preferably, they contain about 0.7 - 0.9% and most preferably, they contain about 0.75%. Amounts lower than about 0.5 weight percent are not preferable in that the emulsifiers would not be effective to emulsify the compositions at such low quantities. Amounts higher than about 1.2 weight percent would cause the composition to form a gel, which would be an unacceptable form for cleansing compositions.

The compositions of this invention also preferably include a water-insoluble oil. As used herein, the term "water-insoluble oil" refers to an oil that has water solubility of less than one percent. Suitable water insoluble oils include, but are not limited to mineral oils, petrolatum, vegetable oils, nut oils, fruit oils, seed oils, glyceryl esters of fatty acids, triglycerides, waxes and other mixtures of esters such as isostearyl isostearate and the like, known to those of skill in the art, polyethylene and non-hydrocarbon based oils such as dimethicone, silicone oils, silicone gums, and the like. Preferred water insoluble oils include mineral oil, petrolatum and combinations thereof.

The amount of water insoluble oil present in the compositions of this invention preferably range from about 0.5 percent to about 4 percent, more preferably from about 3 percent to about 3.5 percent and most preferably about 3.25 percent. If the amount of oil present in compositions were to be lower than about 0.5 weight percent, the compositions would be incapable of functioning as cleansers. If the amount of oil were higher than about 4 weight percent, the composition would be overly thick and have an unacceptable "afterfeel" when used on the skin.

The compositions of this invention preferably further include one or more fatty alcohols. As used herein, the term "fatty alcohol" means a linear or branched alcohol having from 14 to 22 carbon atoms. Preferred fatty alcohols include, but are not limited to, cetyl alcohol, cetearyl alcohol, myristyl alcohol, pentadecyl alcohol, stearyl alcohol, oleyl alcohol and mixtures thereof. The amount of fatty alcohol preferably ranges from about 1 percent to about 4 percent of the composition, more preferably, from about 2.5 percent to about 3.5 percent and most preferably, 3 percent. If the fatty alcohol were present in an amount less than about 1 weight percent, the composition would not function well as a cleanser. In an amount higher than about 4 weight percent, the composition would be similar to a lotion, which would be aesthetically unacceptable to users. Preferably, the ratio between the weight percent of fatty alcohol present in the compositions of this invention and the weight percent of insoluble oil should be about 2:1.

Cleansing compositions should impart to the user a clean, fresh experience. In order to achieve this effect, the cleansing composition should have a high water content. Similarly, it is undesirable to have a heavy or greasy feel to a personal care cleanser. In order to avoid a heavy or greasy feel it is likewise important to maintain a high water content. Notwithstanding the "creamy texture" of the compositions of this invention, they should also have such a clean, fresh feel. The compositions of this invention preferably contain greater than about 92 percent, more preferably, greater than about 93 percent and less than about 98 percent and most preferably, about 93.75 percent of water.

Preferably, the compositions of this invention may also include ingredients useful as foaming agents. For example, such ingredients may be selected from the following materials: hydrophobically modified polyacrylic acid, acrylamidoalkyl sulfonic acid, cyclic N-vinylcarboxamides, acrylamidoalkyl sulfonic acid, hydrophobically modified methacrylic acid, a homopolymer of acrylamidoalkyl sulfonic acid, or combinations thereof as polymeric emulsifiers; and monomeric anionic surfactants, monomeric amphoteric surfactants, or combinations thereof as foaming agents. The compositions that include hydrophobically modified polyacrylic acid; water-soluble or water-swellable copolymers based on acrylamidoalkyl sulfonic acid, cyclic N-vinylcarboxamides; water-soluble or water-swellable copolymers based on acrylamidoalkyl sulfonic acid, hydrophobically modified methacrylic acid; a homopolymer of acrylamidoalkyl sulfonic acid, or combinations thereof as polymeric emulsifiers, may include a betaine as a foaming surfactant.

As used herein and throughout the application, all percents represent percent by weight of the active ingredient (i.e., carriers such as water and impurities are not included in these percentages) based on the total weight of composition, unless otherwise indicated. Also, when it is stated that a particular category of ingredient, e.g., polymeric emulsifier is "present in a weight percentage" that is in a certain range, this implies that the sum total concentration of all ingredients that are classified as polymeric emulsifiers are present in that present range.

The compositions of this invention may also include chelating agents in order to maintain the thickness of the compositions in view of the presence of the polymeric emulsifier component, as this component tends to be sensitive to salt in its environment. Examples of suitable chelating agents include those which are capable of protecting and preserving the compositions of this invention. Preferably, the chelating agent is ethylenediamine tetracetic acid ("EDTA"), and more preferably is tetrasodium EDTA, available commercially from Dow Chemical Company of Midland, Michigan under the tradename, "Versene 100XL" and is present in an amount, based upon the total weight of the composition, from about 0 to about 0.5 percent or from about 0.05 percent to about 0.25 percent. Other suitable preservatives include Quaternium-15, available commercially as "Dowicil 200" from the Dow Chemical Corporation of Midland, Michigan, and are present in the composition in an amount, based upon the total weight of the composition, from about 0 to about 0.2 percent or from about 0.05 percent to about 0.10 percent.

The methods and compositions of this invention illustratively disclosed herein suitably may be practiced in the absence of any component, ingredient, or step which is not specifically disclosed herein. Several examples are set forth below to further illustrate the nature of the invention and the manner of carrying it out.. The scope of the invention is defined by the appended claims.

### Measuring Barrier Function

Transepithelial water loss ("TEWL") and skin hydration constitute two areas of measurements by which to determine skin function. However, absolute measurements generated by these test methods may require additional means by which to understand the characteristics and extent of barrier impairment. For example, two different people may be exposed to the environment but present very different TEWL or skin hydration measurements from their exposed skin depending on the nature of their own particular skin properties. Likewise, different environments may produce similar TEWL or skin hydration measurements in very different people. Therefore, when determining the effect of the application of the compositions of this invention to skin it is best to examine how the TEWL or skin hydration level may change upon exposure to cleansing compositions and measure the change in TEWL or skin hydration after exposure.

The hydration level of the stratum corneum affects its mechanical and electrical properties, thus the Ski-Con-200EX (I.B.S Co., LTD., Japan), which measures the high frequency conductivity of the skin, can be used to measure the relative water holding capacity of the superficial corneocytes (first layer). The measurement may be performed by placing a probe on the skin surface for a period of time. The probe is connected to a computer or other data recording device. Skin hydration measured via conductance is expressed as micro Siemans, "µS".

### Skin Barrier Mildness Test

In this Test, adult subjects (aged 18-65) who were diagnosed with atopic dermatitis (n=25 subjects/cell) were exposed to cleansing solutions set forth in Table 1 (diluted as defined below) on their volar forearms under an occlusive patch for 24 hours. After 24 hr, the patch and cleansing solution was removed and a new patch with the same treatment was reapplied, and this was repeated for a total of four days. The skin barrier function was measured in accordance with the measurement of TEWL before treatment and then after the 4 days of patching. The TEWL reported was the change in the TEWL at Day 4 from baseline (i.e., before patching of the cleansing composition).

In this test, compositions are diluted by 50% prior to placement on the skin to measure TEWL. The data set forth in Table 2 below demonstrate the change in TEWL (Delta TEWL), as assessed at Day 4, in comparison to Day 1, before exposure to 50% dilution in distilled water of the cleansing composition.

### Skin Irritancy Test

*In vitro* Skin equivalents have been validated as a human skin model, and have effectively demonstrated a correlation between in vitro and in vivo effects of surfactants on skin as well as other consumer products. The EpiDerm™ Skin Model provided by MatTek Corporation was used in this study. The target cells were epithelial, derived from human skin. The test materials were applied directly to the culture surface, at air interface, so that undiluted and/or end use dilutions were tested directly. The experimental design used in this study consisted of a definitive assay to determine the release of one cytokine where tissue viability was not decreased by 50% as compared to the negative control tissue (as measured by MTT reduction), the inflammatory potential was then measured by the synthesis/release of the cytokine IL-1α.

In the treatment phase, six skin equivalents (hereinafter, "Tissue") were used for each diluted test product, individual results were averaged to provide an overall response. 100µl was applied to each equivalent for 1 hour of diluted product (10% dilution) exposure followed by 5 rinses of Ca, Mg Free PBS solution. Each Tissue was placed in a 6 well tray with assay medium for each rinse and returned to incubation for 24 hours. Following incubation, Tissues were assessed for cytokine responses in IL-1α.

### Emulsion Particle Size Test

Emulsion particle size was measured using an Olympus BX51 light microscope. Drops of samples of compositions of this invention were placed on a glass slide and covered with a glass cover slip. The slide was then tapped lightly to spread the sample. All the microscopy measurements were performed at room temperature in transmission. An image was obtained through the microscope, after which the particle size of at least 20 droplets was measured and averaged. The average oil phase droplet size is reported in Table 7.

### Cleansibiliy Test 1 (Mascara)

Vitro Skin (IVS, MA) was used as a test substrate. A test area the size of 2.5 cm² was marked on the Vitro Skin substrate. Mascara (Maybelline Great Lash, Very Black) was applied to the Vitro Skin substrate as the test material to determine whether it could be removed from the Vitro Skin substrate using the compositions of this invention. 50µL of mascara was applied in a dot pattern evenly over the Vitro Skin substrate. A cotton swab was then used to spread the mascara over the test area, and the sample allowed to dry for 30 minutes. The test site was then wetted and a baseline measurement was taken with a digital camera. After this step, 0.1 mL of the test cleanser or control composition was applied onto the site and rubbed continuously for 30 s. Each test site was then gently rinsed with water for 20 s so as to avoid significant mechanical removal due to water flow. After the first wash cycle, an image was taken of the test site, then the wash and rinse was repeated 3 more times. After the fourth wash, an additional image of the test site was captured.

A common method used to characterize color is Lab color space as taught by Hunter, Richard Sewall (July 1948). "Photoelectric Color-Difference Meter". JOSA 38 (7): 661. (Proceedings of the Winter Meeting of the Optical Society of America), which is incorporated herein by reference. This method was designed to approximate human vision and is used herein to measure how well the compositions of this invention act to cleanse by comparing the lightness of a treated sample with the image of said sample prior to exposure to cleansing compositions. "L" is a measure of lightness, and "a" and "b" are the color components of the image. A high L value indicates a light image, and a low L value indicates a darker image. In this test, a Vitro Skin substrate without mascara was initially largely colorless and had a high L value. The addition of mascara rendered the surface dark, resulting in a low L value. Cleansing scores were calculated as follows:

| | |
|---|---|
| ΔL1: | = L (after 1 wash) - L(before washing) |
| ΔL4: | = L (after 4 wash) - L(before washing) |
| ΔL 1w: | = L (after 1 wash) - L(after 1 wash with water) |
| ΔL 4w: | = L (after 4 wash) - L(after 4 wash with water) |

### Cleansibiliy Test 2 (Diaper rash cream)

Vitro Skin (IVS, MA) was used as a test substrate. A test area the size of 2.5 cm² was marked on the Vitro Skin substrate. White Diaper rash cream was used as the test material to be applied to the Vitro Skin substrate. 50µL of diaper rash cream (Desitin Original Zinc Oxide Diaper Rash Ointment) was applied in a dot pattern evenly over the Vitro Skin substrate. A cotton swab was then used to spread the diaper rash cream over the test area and the treated material allowed to dry for 30 minutes. The test site was then wetted and a baseline measurement was collected with a digital camera. The test cleanser in an amount of 0.1 mL was applied to the site and rubbed continuously for 30 s. Each test site was then gently rinsed with water for 20 s so as to avoid significant mechanical removal due to water flow. After the first wash cycle, an image was taken of the test site, then the wash and rinse cycle was repeated 3 more times. After the fourth wash, an image of the test site was taken. The images were then scored by people as to the extent of material removed, or the extent of cleansing. The cleansiblity scale was as follows: 1 = no removal of diaper rash cream; vitro skin appears the same as before cleansing; 5 = complete removal of diaper rash cream, vitro skin is completely cleaned, same as before diaper rash application.

### Example 1

Cleansing compositions of Examples E1, E2, and C3 - C6 were prepared in accordance with the procedure set forth below with the ingredients set forth in Table 1.

**Table 1***

| | **E1** | **E2** | **C3** | **C4** | **C5** | **C6** |
|---|---|---|---|---|---|---|
| **INCI Name** | (wt%) | (wt%) | (wt%) | (wt%) | (wt%) | (wt%) |
| **Water phase** | | | | | | |
| Water | 93.2 | 93.8 | 95.7 | 97.7 | 95.7 | 96.2 |
| Ammonium Acryloyldimethyltaurate /VP Copolymer | 1.00 | 0.4 | 0.5 | 0.5 | 0.5 | - |
| Glycerin | 1.00 | 1.00 | 1.0 | 1.0 | 1.0 | 1.00 |

| **Oil Phase** | | | | | | |
|---|---|---|---|---|---|---|
| Mineral Oil | 0.75 | 0.75 | - | - | 1.5 | - |
| Petrolatum | 0.25 | 0.25 | - | - | 0.5 | - |
| Cetyl Alcohol | 3.00 | 3.00 | 2.00 | - | - | 2.0 |
| Post Phase | | | | | | |
| Methylparaben | 0.2 | 0.2 | 0.2 | 0.2 | 0.2 | 0.2 |
| Phenoxyethanol | 0.6 | 0.6 | 0.6 | 0.6 | 0.6 | 0.6 |
| | | | | | | |
| Total oil phase | 4.00 | 4.00 | 2.00 | 0.00 | 2.00 | 2.00 |
| Total water phase | 96.00 | 96.00 | 98.00 | 100.00 | 98.00 | 98.00 |

| | | | | | | |
|---|---|---|---|---|---|---|
| *expressed in active % w/w | | | | | | |

Each of the compositions of Table 1 was independently prepared as follows: Water (50.0 parts) was added to a beaker. Glycerin and Ammonium Acryloyldimethyltaurate/VP Copolymer were then added thereto independently with mixing until each respective resulting mixture was homogeneous. The water phase was then heated to about 60° C. The oil phase was then prepared in a second vessel by mixing Mineral Oil, Petrolatum, and Cetyl Alcohol therein and the mixture heated to about 60° C. The oil phase was then slowly added to the water phase while under agitation. The remainder of the ingredients, Methylparaben and Phenoxyethanol, and the remainder of the water were then added thereto.

### Skin Barrier Mildness Test

The data set forth in Table 2 below demonstrate the change in TEWL (Delta TEWL), as assessed at Day 4 of usage in the TEWL test (as described above), in comparison with Day 1, before exposure to 50% dilution in distilled water of the cleansing composition. Comparative Sample C7 was the creamy cleanser Cetaphil® Gentle Skin Cleanser (Galderma, Fort Worth,TX). Cetaphil® is considered a gentle cleanser appropriate for patients with an impaired skin barrier, and is commonly recommended by dermatologists for patients with this condition. Cetaphil's ingredients are listed as follows: Water, Cetyl Alcohol, Propylene Glycol, Sodium Lauryl Sulfate, Stearyl Alcohol, Methylparaben, Propylparaben, Butylparaben. C8 was Purpose® Gentle Face Cleanser from Johnson & Johnson Consumer Companies, Inc. (Skillman, NJ). C9 was Neutrogena® Fresh Foaming Cleanser, available from Neutrogena Corporation (Los Angeles, CA). C10 was Aveeno® Ultra-Calming Foaming Cleanser, available from Johnson & Johnson Consumer Companies, Inc. (Skillman, NJ).

**Table 2**

| | Cleansing composition | DELTA TEWL (Day 4 - baseline) (g/m²/hr) |
|---|---|---|
| C7 | Cetaphil Gentle Skin Cleanser | 8.7 |
| C8 | Purpose Gentle Cleanser | 7.2 |
| C9 | Neutrogena Fresh Foaming Cleanser | 6.3 |
| C10 | Aveeno Ultra-Calming Foaming Cleanser | 5.2 |
| E1 | Composition of the invention | 2.7 |
| | Water | 1.7 |
| | No treatment (patch only) | 0.9 |

As set forth in Table 2, the results demonstrate that exposure to a blank patch without any cleansing composition or to water alone resulted in a very small increase in TEWL, thus a small Delta TEWL value. A small Delta TEWL value demonstrates no significant change in the skin barrier after treatment with water. For cleansing compositions that are mild to the skin, the Delta TEWL was also small, indicating minimal interaction between the cleansing composition with the skin. E1 was also shown to have a smaller Delta TEWL as compared with commercial mild skin cleansers that were tested. Comparative examples, C8 - C9, were mild foaming surfactant based cleansers.

### Skin Irritancy Test

The data set forth in Table 3 below demonstrate the skin irritancy of different cleansing compositions, including compositions made in accordance with this invention as well as comparative compositions. C11 was Olay Foaming Face Wash Sensitive Skin available from Proctor and Gamble (Cincinnati, OH). C12 was Dove Sensitive Skin Foaming Wash Facial Cleanser available from Unilever Inc (Trumbull, CT). C13 was Neutrogena® Oil Free Acne Wash, available from Neutrogena Corporation (Los Angeles, CA).

**Table 3**

| | Cleanser Formula | IL - 1α | St dev (+/-) |
|---|---|---|---|
| | | (pg/ml) | (pg/ml) |
| C11 | Olay Foaming Face Wash Sensitive Skin | 343 | 10 |
| C12 | Dove Sensitive Skin Foaming Wash | 316 | 18 |
| C13 | Neutrogena Oil Free Acne Wash | 262 | 76 |
| C8 | Purpose Gentle Cleanser | 175 | 6 |
| C7 | Cetaphil Gentle Skin Cleanser | 14.4 | |
| E1 | Composition of the invention | 6.7 | |

The data above demonstrate that compositions made according to this invention are very mild.

### Cleansibility - ability to remove unwanted material from the skin surface:

An important aspect of a skin cleanser is its ability to remove unwanted material from the skin, i.e., the ability of the skin cleanser to provide cleaning utility. For a facial cleanser, the ability to remove mascara from the eye area is important. Table 4A below shows the results of the Cleansibility test 1 (Mascara) for different cleansing formulations. C14 was Eucerin Redness relief Soothing Cleanser, available from Beirsdorf (Wilton, CT). Table 4B below shows the results of the Cleansibility test 1 (Mascara) for different cleansing formulations compared to water, the ΔL 1w, and ΔL 4w, as described above.

**Table 4A**

| | | 1 wash (L) | 4 wash (L) | 1 wash (ΔL) | 4 wash (ΔL) |
|---|---|---|---|---|---|
| | | L1 | L4 | ΔL1 | ΔL4 |
| E1 | Polymer, Fatty Acid, Oil | 113 | 135 | 96 | 118 |
| C3 | Polymer, Fatty Acid | 84 | 107 | 67 | 90 |
| C4 | Polymer | 22 | 47 | 5 | 30 |
| C5 | Polymer, Oil | 19.7 | 37 | 2.7 | 20 |
| C6 | Fatty Acid | 21.6 | 39.7 | 4.6 | 23 |
| C7 | Cetaphil® Gentle Skin Cleanser | 23.8 | 39.5 | 6.8 | 23 |
| C10 | Aveeno® Foaming Cleanser | 24.3 | 72 | 7.3 | 55 |
| C14 | Eucerin | 24 | 59.2 | 7.0 | 42 |
| | Water | 23 | 38 | 6 | 21 |

**Table 4B**

| | | ΔL 1w (L) | ΔL 4w (L) |
|---|---|---|---|
| E1 | Polymer, Fatty Acid, Oil | 90 | 97 |
| C3 | Polymer, Fatty Acid | 61 | 69 |
| C4 | Polymer | -1.0 | -1.0 |
| C5 | Polymer, Oil | -3.3 | 1.7 |
| C6 | Fatty Acid | -1.4 | |
| C7 | Cetaphil® Gentle Skin Cleanser | 0.8 | 1.5 |
| C10 | Aveeno® Ultra-Calming Foaming Cleanser | 1.3 | 34 |
| C14 | Eucerin | 1.0 | 21.1 |
| | Water | na | na |

The cleansibility data demonstrate that with respect to the effective cleansing composition made in accordance with this invention, E1, the L value increases from the 1^{st} wash (1 wash) to the 4^{th} wash (4 wash) indicating that more material is removed from the 1^{st} wash to the 4^{th} wash. As the black mascara is removed and the light substrate is revealed, the L value increases as the images change from black to white with washing. The ΔL shows the change from the initial unwashed state, and the ΔL 1W and ΔL 1W show the difference from cleansing with water. Preferably the creamy cleanser compositions according to this invention should have a ΔL 4w score greater than 75.

Example E1 has better cleansibility in accordance with this test than the commercial product comparisons C7, C10, and C14 after both one wash and after four washes, and much superior cleansing compared with water. Without any lipophilic components, water provides very little cleansing. Example E1 containing water, a water insoluble oil, a fatty acid, and a polymeric emulsifier has excellent cleansibility as indicated with a large ΔL 4w. Comparative example, C4, containing only water and the polymeric emulsifier, has poor cleansibility similar to water. Comparative example, C5, containing polymeric emulsifier, water insoluble oil and water also has poor cleansibility similar to water. Comparative example, C6, containing fatty acid and water also has poor cleansibility similar to water. Comparative example, C3, containing polymeric emulsifier and fatty acid and water has fairly good cleansibility, similar to water. However C3 has much lower cleansibility than Example E1, which also contains water insoluble oil.

### Cleansibility Test 2 (Diaper rash cream)

The results of the Cleansibility Test 2 (Diaper Rash Cream) for different cleansing formulations are set forth in Table 5. As described above, the cleansiblity scale was: "1" no removal of diaper rash cream to "5" complete removal of diaper rash cream.

C15 was Axe Revitalizing Shower Gel, Phoenix available from Unilever Inc. C16 was Johnson's Head-To-Toe Baby Wash^{®} available from Johnson & Johnson Consumer Companies, Inc. (Skillman, NJ).

**Table 5**

| | | Cleansibility (score) |
|---|---|---|
| E1 | Composition of the invention | 4.78 |
| C7 | Cetaphil® Gentle Skin Cleanser | 1.93 |
| C15 | Axe Body Wash | 4.14 |
| C16 | Johnson's Head-To-Toe Baby Wash^{®} | 3.21 |
| | Water | 1.90 |

The data in Table 5 demonstrates that the compositions of this invention provide excellent cleansiblity. The compositions performed better than a harsh or aggressive body wash.

### Example 2

The cleansing compositions of Examples C17, C18, and E19 - E20 were prepared according to process set forth below using ingredients in the amounts stated in Table 6.

**Table 6**

| | **C17** | **C18** | **E19** | **E20** |
|---|---|---|---|---|
| **INCI Name** | (wt%) | (wt%) | (wt%) | (wt%) |
| **Water phase** | | | | |
| Water | Q.S. | Q.S. | Q.S. | Q.S. |
| Ammonium Acryloyldimethyltaurate /VP Copolymer | 0.80 | 0.80 | 0.80 | 0.80 |
| Glycerin | 5.00 | 5.00 | 5.00 | 5.00 |

| **Oil Phase** | | | | |
|---|---|---|---|---|
| Mineral Oil | 3.30 | 2.83 | 2.33 | 2.00 |
| Cetyl Alcohol | 6.60 | 5.66 | 4.66 | 4.00 |
| Post Phase | | | | |
| Methylparaben | 0.2 | 0.2 | 0.2 | 0.2 |
| Phenoxyethanol | 0.6 | 0.6 | 0.6 | 0.6 |
| | | | | |
| Total oil phase | 10.0 | 8.5 | 7.0 | 6.0 |
| Total water phase | 90.0 | 91.5 | 93.0 | 94.0 |

Each of the compositions of Table 6 was independently prepared as follows: Water (50.0 parts) was added to a beaker. Glycerin and Ammonium Acryloyldimethyltaurate/VP Copolymer were then added thereto independently, with mixing, until each respective resulting mixture was homogeneous. The water phase was then heated to about 60° C. The oil phase was prepared in a second vessel by mixing Mineral Oil and Cetyl Alcohol. This mixture was then heated to about 60° C. The oil phase was then slowly added to the water phase while under agitation. The remainder of the ingredients (Methylparaben and Phenoxyethanol) and the remainder of the water were then added thereto.

### Particle Size Test

The size of the oil domains in the creamy cleanser emulsion were determined by a Particle size test and the results are shown in Table 7 below.

**Table 7**

| Example | C17 | C18 | E19 | E20 |
|---|---|---|---|---|
| | | | | |
| Oil phase (%) | 10.0 | 8.5 | 7.0 | 6.0 |
| Particles size of oil phase (Δm) | 19.67 | 17.3 | 13.1 | 16.2 |

The particle sizes of the oil phase particles were larger in the formulas with higher weight percentages of oil phase, C17 and C18. These larger oil phase droplets should be more likely to be left behind on the skin after washing with the creamy cleanser. The oil phase that is left behind on the skin is likely to create a greasy or oily feeling on the skin, which is undesirable.

### Skin Feel of Creamy Cleanser - In-use survey data on Skin feel of creamy cleanser:

A home use study was conducted using compositions made in accordance with this invention as well as comparison compositions. Subjects were instructed to use each creamy cleanser at home as a facial cleanser, then to fill out a survey that contained questions related to the skin feel and in use feel of the creamy cleanser. Ten people used each of the creamy cleanser compositions as set forth below. After use, the subjects rated each composition for both the extent of greasiness and the extent of heaviness using the following scales:
Scale of greasiness (1-5): (1: not at all greasy, 5: very greasy),
Scale of heaviness (1-5) (1: not at all heavy, 5: very heavy).

Additionally, each subject was presented with a list of adjectives and asked to select the adjective that they felt described the feel and skin feel of each example creamy cleanser. The list of descriptor words presented to each subjected was as follows: comfortable, clean, creamy, dry, greasy, healthy, heavy, hydrated, irritant, itchy, lubricious, moisturized, oily, residue, rough, scaly, silky, slippery, smooth, squeaky clean, soft, sticky, stiff, supple, tacky, taut, tight, velvety, and waxy. From the list of descriptor words, words that are related to greasiness or heaviness were counted for each formulation (these words include: residue, greasy, oily, waxy, sticky, residue, slippery).

The results are shown in Table 8 below.

**Table 8**

| Example | C17 | C18 | E19 | E20 |
|---|---|---|---|---|
| Oil phase (%) | 10.0. | 8.5 | 7.0 | 6.0 |
| Greasiness (1-5) | 2.8 | 2.8 | 1.8 | 2.0 |
| Heaviness (1-5) | 2.8 | 3.0 | 2.0 | 2.0 |
| Number of descriptors related to greasiness or heaviness | 5 | 6 | 1 | 1 |

The "skin feel", or aesthetics, of a composition that is used on the skin is an important parameter to consider when formulating such compositions, particularly among creamy cleanser-type compositions. Good cleansibility can be achieved with formulas having a very high oil content. However, compositions containing a high percentage of oils tend to feel greasy or otherwise aesthetically unacceptable. For example, pure mineral oil or toluene are both very good at removing hydrophobic materials from the skin, however both offer very poor skin feel in that users would find them to be greasy or heavy. In the skin feel study results set forth above in Table 8, the two examples with high oil phase amounts, C17 and C18, both were found to have poor skin feel due to greasiness and heaviness. Further, when offered a list of words to describe the formulas, study participants described both C17 and C18 with words related to greasiness/heaviness. Thus, the high oil load in C17 and C18 resulted in a formula that has poor skin feel. In contrast with the comparative examples C17 and C18, E19 and E20 made in accordance with this invention had a good skin feel results, as determined by the panel participants. E19 and E20 contained lower oil phase levels and both were found not to have high levels of Greasiness or Heaviness.

### Creamy Cleanser Compositions Examples

The cleansing compositions of Examples E21 - E24 were prepared according to process set forth below using ingredients in the amounts stated in Table 9.

**Table 9**

| | **E21** | **E22** | **E23** | **E24** |
|---|---|---|---|---|
| **INCI Name** | (wt%) | (wt%) | (wt%) | (wt%) |
| **Water phase** | | | | |
| Water | q.s. | q.s. | q.s. | q.s. |
| Ammonium Acryloyldimethyltaurate /VP Copolymer | 0.75 | 0.75 | 0.75 | 0.75 |
| Glycerin | 1.00 | 1.00 | 1.00 | 1.00 |

| **Oil Phase** | | | | |
|---|---|---|---|---|
| Soybean Oil | 0.50 | | | |
| Partially Hydrogenated Cottonseed Oil | 0.50 | | 0.25 | 0.25 |
| Sunflour Seed Oil | | 0.75 | | |
| Partially Hydrogenated Coconut Oil | | 0.25 | | |
| Triticum Vulgare (Wheat) Germ Oil | | | 0.75 | |
| Mauritia Flexuosa Fruit Oil | | | | 0.75 |
| Cetyl Alcohol | 3.00 | 3.00 | 3.00 | 3.00 |

| **Post Phase** | | | | |
|---|---|---|---|---|
| Phenoxyethanol | 1.25 | 1.25 | 1.25 | 1.25 |
| | | | | |
| Total oil phase | 4.0 | 4.0 | 4.0 | 4.0 |
| Total water phase | 96.0 | 96.0 | 96.0 | 96.0 |

Each of the compositions of Table 9 was independently prepared as follows: Water (50.0 parts) was added to a beaker. Glycerin and Ammonium Acryloyldimethyltaurate/VP Copolymer were then added thereto independently with mixing until each respective resulting mixture was homogeneous. The water phase was then heated to about 60° C. The oil phase was prepared by mixing, in a second vessel, Cetyl Alcohol, Soybean Oil, Partially Hydrogenated Cottonseed Oil, Sunflour Seed Oil, Partially Hydrogenated Coconut Oil, Triticum Vulgare (Wheat) Germ Oil, and or Mauritia Flexuosa Fruit Oil as called for and heated to about 60° C. The oil phase was then slowly added to the water phase while under agitation. The remainder of the ingredients (Phenoxyethanol) and the remainder of the water were then added thereto.

## Claims

1. A creamy cleansing composition comprising:
(a)from 0.5 % to 1.2 % polymeric emulsifier;
(b)from 0.5 % to 4 % water insoluble oil;
(c)from 1 % to 4 % fatty alcohol, wherein the fatty alcohol is a linear or branched alcohol having from 14 to 22 carbon atoms; and
greater than 92 % water.

2. A creamy cleansing composition according to claim 1 wherein said polymeric emulsifier is salt-sensitive.

3. A creamy cleansing composition according to claim 2 wherein said polymeric emulsifier comprises monomers comprising hydrophilic repeat units.

4. A creamy cleansing composition according to claim 3 wherein said monomers are selected from the group consisting of acrylic acid, methacrylic acid, maleic acid, itaconic acid and combinations thereof.

5. A creamy cleansing composition according to claim 2 wherein said polymeric emulsifier comprises a polysaccharide.

6. A creamy cleansing composition according to any one of claims 2 to 5 wherein said polymeric emulsifier comprises hydrophilic groups selected from the following moieties: hydroxyl, carboxyl, sulphate, sulphonate, taurate, phosphate, phosphonate, amide, amine and a combination thereof.

7. A creamy cleansing composition according to any preceding claim wherein said polymeric emulsifier is at least partially hydrophobically modified.

8. A creamy cleansing composition according to claim 7 wherein said polymeric emulsifier is hydrophobically modified with one or more hydrocarbons having a carbon chain length of at least three carbon atoms.

9. A creamy cleansing composition according to claim 8 wherein said polymeric emulsifier is selected from the group consisting of hydrophobically modified polyacrylic acid, water-soluble copolymers based on acrylamidoalkyl sulfonic acid and cyclic N-vinylcaboxamides, water-swellable copolymers based on acrylamidoalkyl sulfonic acid and cyclic N-vinylcaboxamides, water-soluble copolymers based on acrylamidoalkyl sulfonic acid and hydorphobically modified methacrylic acid, water-swellable copolymers based on acrylamidoalkyl sulfonic acidand hydorphobically modified methacrylic acid and homopolymer of acrylamidoalkyl sulfonic acid and a mixture thereof.

10. A creamy cleansing composition according to any preceding claim wherein the ratio of fatty alcohol to water insoluble oil present in said composition is 2:1.

11. A creamy cleansing composition according to any preceding claim wherein said water-insoluble oil is selected from the group consisting of mineral oil, petrolatum, vegetable oils, fruit oils, nut oils, seed oils, glyceryl esters of fatty acids, triglycerides, waxes, polyethylene, silicone oils, silicone gums and mixtures thereof.

12. A creamy cleansing composition according to claim 11 wherein said water-insoluble oil is mineral oil.

13. A creamy cleansing composition according to any preceding claim wherein said fatty alcohol is selected from the group consisting of cetyl alcohol, cetearyl alcohol, myristyl alcohol, pentadecyl alcohol, stearyl alcohol, oleyl alcohol and mixtures thereof.

14. A creamy cleansing composition according to claim 13 wherein said fatty alcohol is cetyl alcohol.

15. A creamy cleansing composition according to any preceding claim wherein the amount of water insoluble oil present in said composition is from 3 weight percent to 3.5 weight percent.

16. A creamy cleansing composition according to any preceding claim wherein the amount of water insoluble oil present in said composition is 3.25 weight percent.

17. A creamy cleansing composition according to any preceding claim wherein the amount of fatty alcohol present in said composition is from 2.5 weight percent to 3.5 weight percent.

18. A creamy cleansing composition according to claim 17 wherein the amount of fatty alcohol present in said composition is 3 weight percent.

19. A creamy cleanser composition according to any preceding claim wherein said composition contains at least 93 weight percent of water.

20. A creamy cleanser composition according to claim 19 wherein said composition contains at least 93.75 weight percent of water.

21. A creamy cleanser composition according to any preceding claim wherein its ΔL 4w score is greater than 75.
